# EUROPÄISCHE PATENTSCHRIFT

(11) **EP 2 777 583 B1**
(45) Veröffentlichungstag und Bekanntmachung des Hinweises auf die Patenterteilung: **01.07.2020**
(21) Anmeldenummer: 13159361.8
(22) Anmeldetag: 15.03.2013
(51) Int. Cl.: A61B 18/14, A61B 17/32

(54) **Instrument zur Gefäßfusion und Trennung**
Instrument for vessel fusion and separation
Instrument de fusion et de séparation de récipients

(43) Veröffentlichungstag der Anmeldung: 17.09.2014
(73) Patentinhaber: Erbe Elektromedizin GmbH, 72072 Tübingen (DE)
(72) Erfinder: Düppuis, Martina, 72124 Pliezhausen (DE)
(74) Vertreter: Rüger Abel Patentanwälte PartGmbB

(56) Entgegenhaltungen:
- EP-A1- 1 810 625
- EP-A1- 2 554 133
- WO-A1-95/15124
- WO-A1-2008/124271
- US-A1- 2011 087 221

## Beschreibung

Die Erfindung betrifft ein Instrument zum Fassen, Koagulieren und Durchtrennen von Geweben, insbesondere zum Abklemmen, Verschließen und Durchtrennen von Gefäßen, insbesondere Blutgefäßen, am lebenden Körper eines menschlichen oder tierischen Patienten.

Instrumente zum Verschließen und Trennen von Blutgefäßen sind beispielsweise der DE 602 26 015 T2 oder EP 2 554 133 A1 zu entnehmen.

Typischerweise weisen solche Instrumente einen länglichen Schaft auf, der sich von einem Griff weg erstreckt. An dem distalen Ende des Schafts ist ein Werkzeug mit zwei Branchen zum Abklemmen eines Blutgefäßes angeordnet. Außerdem ist dort ein längs verschiebbares Messer angeordnet, um ein gefasstes und koaguliertes Gefäß durchtrennen zu können. Die Branchen und das Messer sind über entsprechende Betätigungsorgane an dem Handgriff zu betätigen. Die Branchen sind als Elektroden ausgebildet und gezielt bestrombar, um das zwischen ihnen geklemmte Gefäß zu erwärmen und die Gefäßwände durch Koagulation zu fusionieren.

Vor der Durchtrennung von Blutgefäßen muss sichergestellt werden, dass die Koagulation in ausreichendem Maße stattgefunden hat, so dass die Enden des durchtrennten Gefäßes verlässlich geschlossen bleiben.

Davon ausgehend ist es Aufgabe der Erfindung, ein Instrument zur Gefäßfusion und Trennung zu schaffen, mit dem sich Gefäße verlässlich verschließen und durchtrennen lassen.

Diese Aufgabe wird mit dem Instrument nach Anspruch 1 gelöst:
Das erfindungsgemäße Instrument weist ein Werkzeug mit zwei Branchen auf, von denen wenigstens eine beweglich ist, so dass sie aufeinander zu und voneinander weg bewegbar sind. Die beiden Branchen bilden ein Maul beziehungsweise ein Mittel zum Greifen von Gewebe, das sich über ein entsprechendes sich durch den Schaft erstreckendes Übertragungselement, beispielsweise einen Zugdraht, schließen lässt. Zu dem Werkzeug gehört weiter ein Messer, das in Ruheposition außerhalb eines zwischen den Branchen definierten Raumes steht und durch ein Antriebselement in diesem Raum eingeführt werden kann. Das Messer besteht vorzugsweise aus einem elektrisch leitfähigen Material, zum Beispiel Edelstahl. Das Antriebselement kann ebenfalls aus einem elektrisch leitfähigen Material, zum Beispiel Edelstahl, ausgebildet sein. Zur Betätigung der beweglichen Branche(n) und des Messers sind an dem Gehäuse ein oder mehrere Betätigungsorgane angebracht, die dazu dienen, zunächst das Maul zu schließen und dann das Messer in das Maul einzufahren. Zur Verbindung des Messers mit dem Antriebselement ist ein Isolator vorgesehen, der eine mechanische Verbindung zwischen dem Antriebselement und dem Messer erbringt, ohne einen Strompfad zwischen beiden zuzulassen. Damit wird die Ausbildung eines parasitären Strompfades unterbunden, der sonst entstehen könnte, wenn das Messer während des Koagulationsprozesses womöglich durch versehentliche Fehlbedienung schon etwas aus seiner Ruhelage heraus zwischen die Branchen bewegt wird.

Die erfindungsgemäße Maßnahme führt auch dazu, dass das Instrument in unterschiedlichen Milieus, beispielsweise auch in Ansammlungen physiologischer Kochsalzlösung oder ähnlichen elektrisch leitfähigen Fluiden, genutzt werden kann. Die Isolation des Messers von dem Antriebselement verhindert bzw. reduziert die Möglichkeit parasitärer Strompfade innerhalb des Werkzeugs oder Schafts des Instruments und stellt somit sicher, dass der auf das biologische Gewebe zu applizierende Strom tatsächlich seinen Weg durch das biologische Gewebe nimmt. Damit wird die gewünschte Intensität des Koagulationsprozesses sichergestellt. Insbesondere wird aber ermöglicht, dass die Bestromung der Branchen vorgenommen und/oder fortgesetzt werden kann, wenn das Messer aus seiner Ruheposition in die Aktivposition bewegt wird. Es wird vermieden, dass über das Messer ein Kurzschluss zwischen den Branchen bzw. ihren Elektroden kommt. Es wird verhindert, dass elektrischer Strom über das Messer an dem Gewebe vorbeigeführt wird. Der elektrische Strom wird von einer Elektrode über das geklemmte Gewebe zu der jeweils anderen Elektrode geleitet. Damit wird die Koagulation des geklemmten Gewebes positiv beeinflusst und auch dann sichergestellt, wenn der Anwender das Messer vorzeitig betätigt. Dies ist insbesondere bei zweistufigen Werkzeugantrieben denkbar, bei denen ein Betätigungsgriff dazu genutzt wird, auf den ersten Teil seines Wegs die Branchen zu schließen und auf einen zweiten, letzten Wegstück das Messer vorzuschieben. Bewegt der Anwender den Handhebel gegebenenfalls unbewusst zu weit in Richtung der Betätigungsposition des Messers, kann dieses schon etwas bewegt werden und somit zumindest einen Teil des Koagulationsstroms übernehmen. Die erfindungsgemäße Isolation des Messers vermeidet diese Fehlermöglichkeit.

Die erste Elektrode weist vorzugsweise eine Messergleitfläche auf, an der das Messer entlanglaufen kann. Auf diese Weise entsteht ein elektrischer Kontakt zwischen der ersten Elektrode und dem Messer, zumindest während des Durchtrennens des koagulierten Gefäßes. Es kann sich somit ein Stromfluss zwischen dem koagulierten Gewebe und dem Messer ergeben, der die Koagulation negativ beeinflussen kann.. Die Isolation des Messers vermeidet diesen Stromfluss sowie auch eine Schädigung des Messers oder seiner Schneidkante oder Spitze durch Stromfluss.

Dem Messer kann ein Vertikalstellmechanismus zugeordnet sein, um das Messer in Abhängigkeit von seiner Längsbewegung quer vorzugsweise senkrecht zu dieser Bewegung zu verstellen, um die Spitze des Messers schwebend zu führen und nur mit der ersten Elektrode nicht aber mit sonstigen Kanten, Stufen oder dergleichen, in Berührung zu bringen. Dies schont das Messer und sorgt für seine anhaltende Schärfe auch bei wiederholter Benutzung.

Die beiden Elektroden weisen vorzugsweise eine zueinander komplementäre Form auf, so dass sie im geschlossenen Zustand zwischen einander einen Spalt von im Wesentlichen konstanter Weite festlegen. Dieser Spalt kann eben oder gewölbt ausgebildet sein. Das Messer ist derart angeordnet und geführt, dass es diesen Spalt durchquert. Vorzugsweise läuft es dabei innerhalb einer der beiden Branchen, die dazu geschlitzt ausgebildet ist, während es auf der Elektrode der anderen Branche gleitet.

Das Messer ist vorzugsweise als Lamelle, d.h. als dünne Metallplatte ausgebildet. Beispielsweise ist es aus einem 0,1 mm bis 0,2 mm starken härtbaren Klingenstahl gefertigt. Es kann eine Länge von einigen Millimetern, beispielsweise 7 mm bis 10 mm und eine Höhe von wenigen Millimetern, beispielsweise 2 mm bis 3 mm aufweisen. Vorzugsweise weist es an seinem distalen Ende eine Schneidnase mit mindestens einer Schneidkante sowie einen sich von der Schneidnase weg erstreckenden Schaft auf. Die Schneidnase und der Schaft können an ein und demselben Metallstück ausgebildet sein und somit nahtlos ineinander übergehen.

Der Isolator ist vorzugsweise ein Kunststoffkörper, in den sich zumindest der Schaft des Messers hinein erstreckt. Dabei bedeckt der Isolator vorzugsweise zumindest die beiden flachen seitlichen Flanken des Schafts und verhindert somit, einen großflächigen Kontakt zwischen dem Messer und vorhandenem biologischem Gewebe sowie etwaiger vorhandener Flüssigkeit. Außerdem erstreckt sich der Isolator oberhalb des Schafts des Messers bis zu einem Isolatorsporn, der sich oberhalb der Schneidnase erstreckt. Der Isolator und sein Isolatorsporn sind (gemessen quer zur Bewegungsrichtung des Messers) dicker als das Messer, insbesondere dicker als seine Schneidnase. So wird jeder elektrische Kontakt zwischen dem Messer und der zweiten Branche vermieden, in deren Schlitz das Messer einfahrbar ist. Die Schneidnase ist geschützt in einer Aussparung des Isolators angeordnet.

Es ist zweckmäßig, wenn der Schaft sowie das Antriebselement jeweils mindestens eine Öffnung aufweisen, die von dem Isolator durchsetzt ist. Auf diese Weise wird ein Formschluss zwischen dem Isolator und dem Antriebselement sowie dem Isolator und dem Messer erhalten. Weiter vorzugsweise legen das Messer und das Antriebselement einen Spalt fest, der als Parallelspalt ausgebildet sein kann. Dieser Spalt kann von Material des Isolators gefüllt sein. Vorzugsweise ist dieser Spalt schräg zur Bewegungsrichtung des Messers orientiert. Damit ergibt sich eine mechanisch belastbare Verbindung zwischen dem Antriebselement und dem Messer, über die insbesondere nicht nur Zug- und Druckbewegungen, sondern auch eine vertikal dazu gerichtete Andruckkraft von dem Antriebselement auf das Messer übertragbar sind. Diese Andruckkraft kann das Messer gegen die ungeschlitzte erste Elektrode drücken und somit ein sauberes Durchtrennen des koagulierten Gewebes unterstützen.

Weitere Einzelheiten vorteilhafter Ausführungsformen der Erfindung sind Gegenstand der Zeichnung, der Beschreibung oder von Unteransprüchen. In der Beschreibung eines Ausführungsbeispiels verwendete richtungsbezogene Begriffe, wie oben, unten, obere, untere usw. beziehen sich auf Richtungen in der Zeichnung und sind insoweit nicht einschränkend auszulegen. Es zeigen:
Figur 1 ein erfindungsgemäßes Instrument in schematisierter Darstellung.
Figur 2 das Werkzeug des erfindungsgemäßen Instruments, in perspektivischer Ansicht.
Figur 3 das Werkzeug nach Figur 2, in Querschnitt.
Figur 4 Messer, Isolator und Antriebselement des Werkzeugs nach Figur 3, in ausschnittsweiser Seitenansicht.
Figur 5 bis 7 das Werkzeug mit geschlossenen Branchen in verschiedenen Betätigungsstellungen und
Figur 8 das Messer, das Antriebselement und den Isolator gemäß Figur 4 in Gesamtansicht.

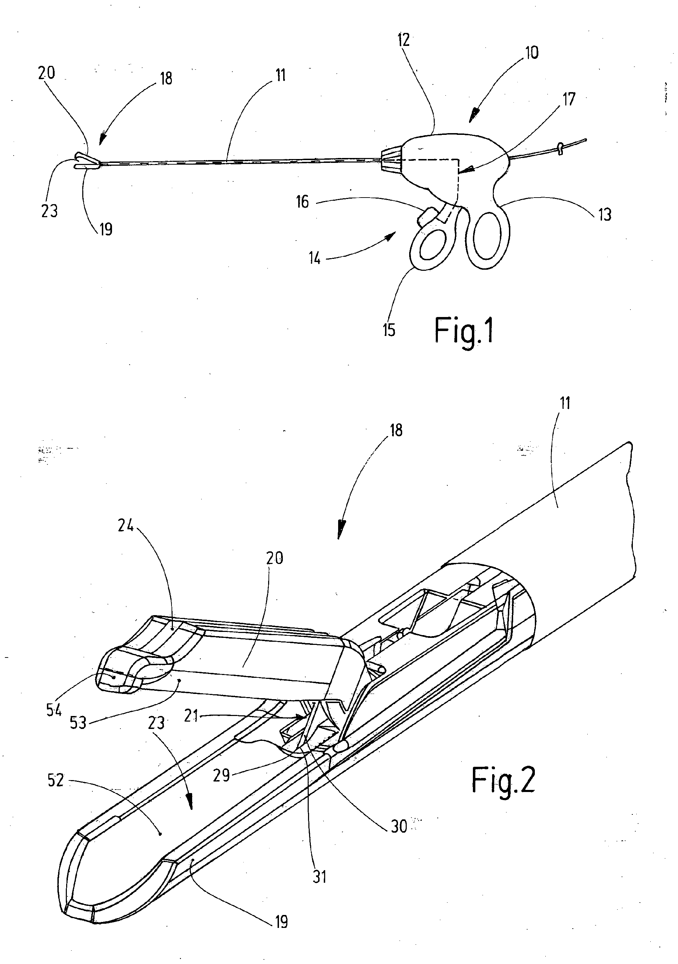

In Figur 1 ist ein Instrument 10 veranschaulicht, das zum Abklemmen, Fusionieren und Durchtrennen von biologischem Gewebe, beispielsweise eines Gefäßes, z.B. eines Blutgefäßes beim Operieren an einem menschlichen oder tierischen Körper dienen kann. Das Instrument 10 kann insbesondere zur endoskopischen Chirurgie eingesetzt werden. Es weist einen schlanken Schaft 11 auf, der an seinem proximalen Ende an einem Gehäuse 12 gehalten ist. Dieses weist ein Handhabungsmittel wie beispielsweise einen Handgriff 13 sowie Betätigungselemente 14 auf. Diese können zum Beispiel aus einem schwenkbaren Griffteil 15 und/oder einem Betätigungsknopf oder Hebel 16 sowie gegebenenfalls weiteren Elementen, z.B. elektrischen Schaltern, bestehen. Die Betätigungselemente 14 sind über in Figur 1 lediglich schematisch gestrichelt angedeutete Kraftübertragungsmittel 17 mit einem an dem distalen Ende des Schafts 11 angeordneten Werkzeug 18 verbunden. Das Werkzeug 18 umfasst mindestens zwei Branchen 19, 20 sowie ein Messer 21. Die Branchen 19, 20 dienen zum Abklemmen von Gewebe, beispielsweise eines Gefäßes, indem sie aufeinander zu bewegt werden, das Gefäß zwischen einander festklemmen und koagulieren. Das Messer 21 dient dazu, das koagulierte Gefäß zu durchtrennen.

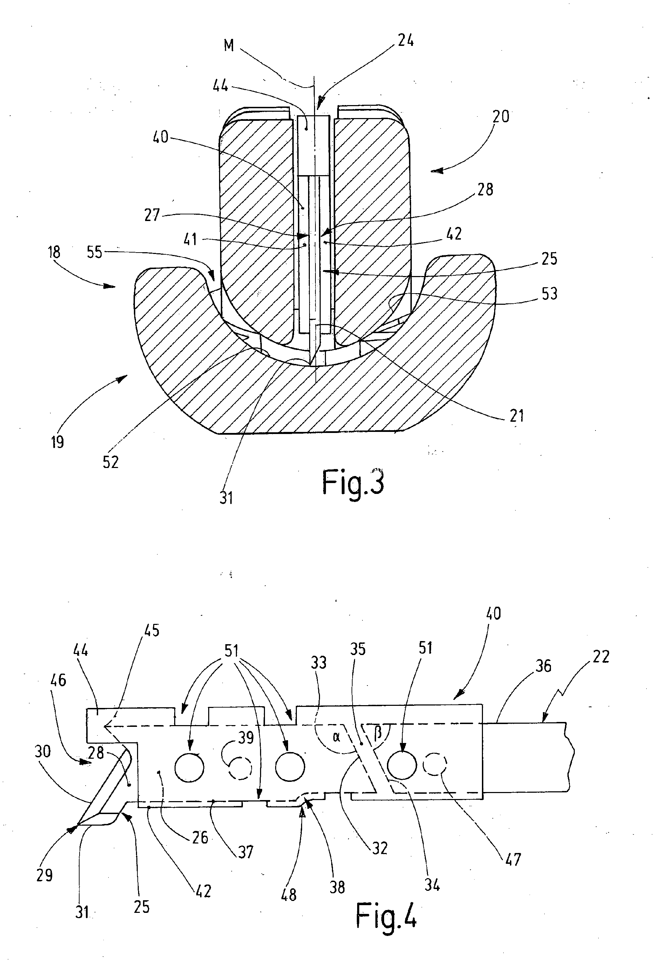

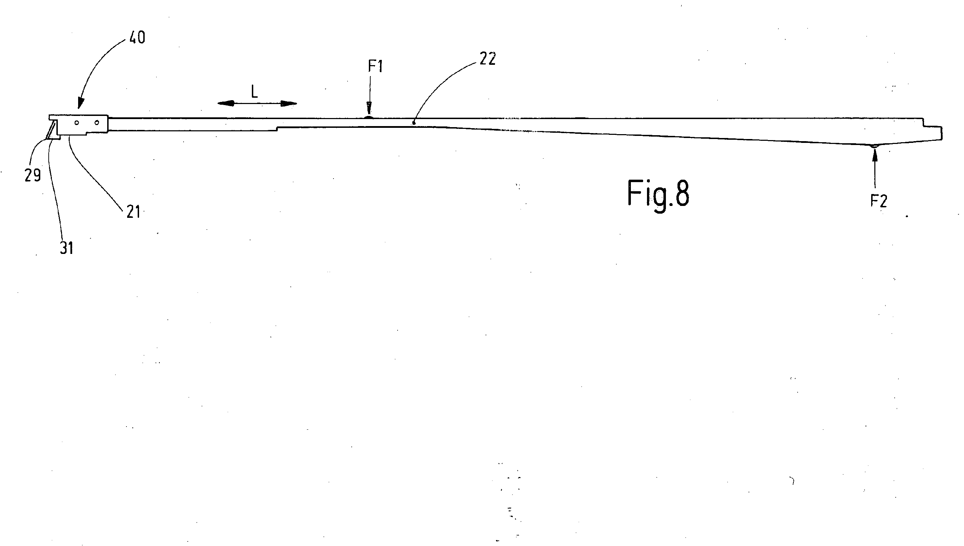

Zu dem Kraftübertragungsmittel können Schub- oder Zugglieder zur Bewegung einer oder beider Branchen 19, 20 sowie ein Antriebselement 22 zur Betätigung des Messers 21 gehören. Ein solches Antriebselement 22 kann beispielsweise ein aus dünnem Blech bestehender Metallstreifen 22 sein, wie er aus Figur 8 ersichtlich ist. Das Antriebselement 22 erstreckt sich von dem Messer 21 durch einen Teilbereich des Schafts 11 bis zu den Schub- und Zuggliedern. Diese erstrecken sich wiederum zu einem nicht weiter veranschaulichten Getriebe, das eine von dem Griffteil 15 ausgehende Antriebsbewegung auf das Messer 21 überträgt. Beispielsweise kann das Getriebe derart ausgebildet sein, dass bei der Bewegung des Griffteils 15 zu dem Handgriff 13 hin zunächst die Branchen 19, 20 geschlossen werden, wobei das Messer 21 noch in Ruhestellung, d.h. außerhalb eines zwischen den Branchen 19, 20 eingeschlossenen Raums 23 bleibt. Dieser Zustand ist in Figur 2 veranschaulicht. Das Getriebe kann weiter so ausgebildet sein, dass auf dem letzten Stück der Bewegung des Griffteils 15 zu dem Handgriff 13 hin bei bereits geschlossenen Branchen 19, 20 das Messer 21 zu dem distalen Ende des Werkzeugs 18 hin zwischen die Branchen 19, 20 vorgeschoben wird. Dazu kann eine der Branchen 19, 20, beispielsweise die Branche 20, mit einem Schlitz 24 versehen sein, der sich längs durch die Branche 20 erstreckt, um das Messer 21 aufzunehmen. Der Schlitz 24 ist darüber hinaus aus Figur 3 ersichtlich. Anstelle des oben beschriebenen Getriebes, das die Bewegung der Branchen 19, 20 und des Messers 21 aus einer Bewegung eines Betätigungsorgans (z.B. Griffteil 15) ableitet, können auch andere, insbesondere getrennte Betätigungsorgane für das Werkzeug 18 und das Messer 21 vorgesehen sein.

Das in dem Schlitz 24 geführte Messer 21 besteht beispielsweise aus einer Metalllamelle, die, wie aus Figur 4 ersichtlich ist, an ihrem distalen Ende eine Schneidnase 25 und einen sich daran anschließenden Schaft 26 aufweist. Die Schneidnase 25 und der Schaft 26 sind vorzugsweise ein Teil eines Metallplättchens von 0,1 mm bis 0,2 mm, vorzugsweise 0,15 mm Dicke, mit zwei Flachseiten 27, 28, die aus Figur 3 hervorgehen. Die Schneidnase 25 bildet eine Klinge mit einer Spitze 29, von der ausgehend sich eine Frontschneidkante 30 schräg ansteigend weg erstreckt. Im spitzen Winkel dazu und damit parallel zur ersten Branche 19 weist die Schneidnase 25 eine Schneidkante 31 auf, die in Benutzung an der ersten Branche 19 gleitet.

Der im Querschnitt rechteckige Schaft 26 erstreckt sich von der Schneidnase 25 weg und endet an einer vorzugsweise gestuften oder auch, wie dargestellt, schräg angeordneten Kante 32. Diese schließt mit der vorzugsweise geraden oberen Kante 33 des Schafts 26, vorzugsweise einen stumpfen Winkel α von zum Beispiel 110° bis 130°, beispielsweise 115°, ein.

Das Antriebselement 22 weist der Kante 32 gegenüber liegend eine stirnseitige Kante 34 auf, die vorzugsweise parallel zu der Kante 32 orientiert ist und dadurch mit dieser einen Parallelspalt 35 festlegt. Die Kante 34 schließt mit der Oberseite 36 des Antriebselements 22 vorzugsweise einen spitzen Winkel β von zum Beispiel 65° ein.

Die sich etwa parallel zu der Oberseite 33 des Schafts 26 erstreckende untere Kante 37 ist vorzugsweise gerade ausgebildet und kann mit einer Stufe 38 versehen sein. Außerdem weist der Schaft 26 vorzugsweise mindestens eine Öffnung 39 auf. Diese kann rund, eckig oder anderweitig ausgebildet sein. Sie dient der formschlüssigen Sicherung eines Isolators 40, der vorzugsweise durch ein Kunststoffteil gebildet ist. Der Isolator 40 hüllt zumindest teilweise den Schaft 26 des Messers 21 ein, wobei der Isolator 40 vorzugsweise die Schneidnase 25 des Messers 21 frei lässt. Der Isolator 40 kann durch ein Kunststoff-Spritzgussteil gebildet sein, vorzugsweise bestehend aus einem temperaturbeständigen Duroplast. Wie aus Figur 3 in Verbindung mit Figur 4 hervorgeht, weist der Isolator vorzugsweise zwei flache Abschnitte 41, 42 auf, die die Flanken 27, 28 des Schafts 26 bedecken und die durch einen sich durch die Öffnung 39 erstreckenden Abschnitt untereinander verbunden sind. Oberhalb der Oberseite des Schafts 26 sowie unterhalb der unteren Kante 37 können die Abschnitte 41, 42 untereinander verbunden sein, so dass insgesamt ein kompakter Isolationskörper ausgebildet ist. Der gemeinsame untere Abschluss der Abschnitte 41, 42 verläuft, wie Figur 4 zeigt, unterhalb der Unterkante des Schafts 26, jedoch oberhalb der Schneidkante 31.

Wie Figur 4 zeigt, ragt das Antriebselement 22 in den Isolator 40 hinein und wird von diesem umhüllt. Vorzugsweise weist der Isolator 40 an seinem distalen Ende einen Isolatorsporn 44 auf, der ein vorderes Ende 45 des Messers 21 umhüllt und über die Schneidnase 25 ragt. Unterhalb des I-solatorsporns ist ein Freiraum 46 ausgebildet, der eine einspringende Ecke mit vorzugsweise einem rechten Winkel bildet. In dem Freiraum 46 ist die Schneidnase 25 angeordnet. Der Isolatorsporn 44 ist gemäß Figur 3 dicker als die Schneidnase 25, die somit die Innenwandflächen des Schlitzes 24 nicht berühren kann.

Es kann in dem Antriebselement 22 eine Öffnung 47 vorgesehen sein, die von Material des Isolators 40 durchsetzt ist, um das Antriebselement 22 und den Isolator 40 formschlüssig zu verbinden. Das Material des Isolators 40 kann außerdem den Parallelspalt 35 ausfüllen.

Der Isolator 40 ist, wie Figur 3 zeigt, an seinen beiden Seiten vorzugsweise flach ausgebildet. Die Abschnitte 41, 42 weisen eine Dicke von 0,1 mm bis 0,3 mm auf. Im Bereich des Schafts 26 beträgt die Materialstärke der Abschnitte 41, 42 z.B. 0,2 mm bis 0,3 mm, vorzugsweise 0,26 mm. Im Bereich des Antriebselements 22 beträgt die Materialstärke der entsprechenden Abschnitte des Isolators 40 vorzugsweise 0,1 mm bis 0,2 mm, z.B. 0,16 mm. Damit wird sowohl eine ausreichende mechanische Stabilität sowie eine gewünschte elektrische Isolation insbesondere der Schneidnase 25 durch ausreichende Beabstandung zu den Flanken des Schlitzes 24 erreicht.

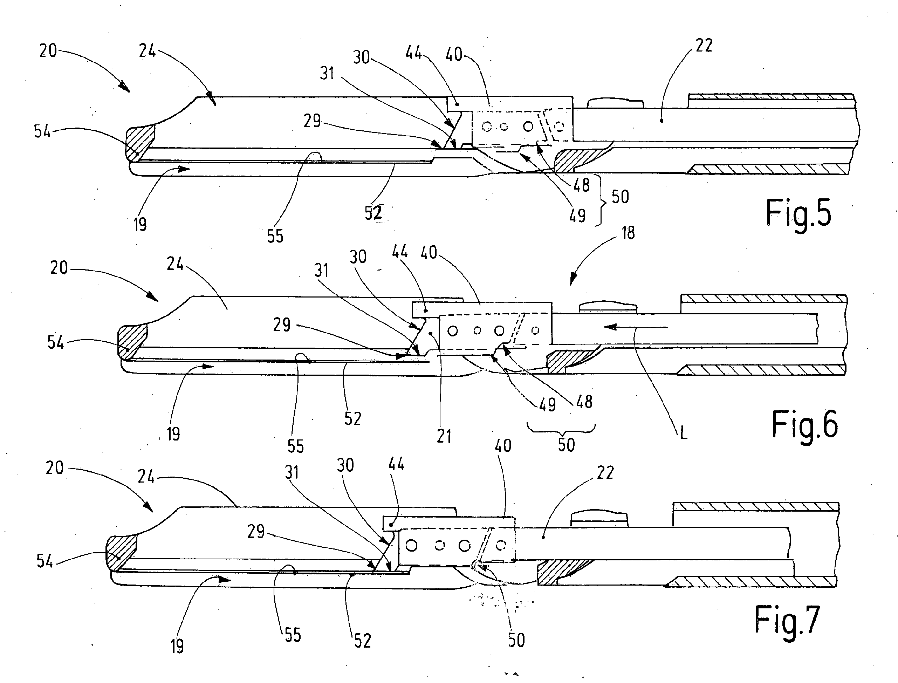

Der Isolator 40 kann an seiner Unterseite bei der Stufe 38 ebenfalls mit einer Stufe 48 versehen sein. Diese bildet zusammen mit einer an der ersten Branche 19 oder dem Schaft 11 vorgesehenen Stufe 49 einen Vertikalstellmechanismus 50 (siehe Figur 5 bis 7). Außerdem kann der Isolator 40, wie Figur 4 zeigt, an mehren Stellen mit Ausnehmungen 51 versehen sein, durch die der Schaft 26 oder auch ein Ende des Antriebselements 22 zugänglich ist. Diese Ausnehmungen 51 können zur Positionierung des Messers 21 und des Antriebselements 22 während der Herstellung des Isolators 40 durch Urformen dienen.

Von dem Isolator 40 ausgehend erstreckt sich das Antriebselement 22 durch den gesamten Schaft 11 und dient dabei zur Bewegung des Messers 21 in Längsrichtung L. Das Antriebeselement 22 ist dazu zug- und drucksteif ausgebildet. Außerdem läuft es in dem Schaft 11 an entsprechenden Gegenflächen, die, wie in Figur 8 durch Pfeile veranschaulicht, positionsabhängig Kräfte F1 und F2 auf das Antriebselement 22 ausüben. Diese Kräfte werden durch den Isolator 40 auf das Messer 21 übertragen, so dass dessen Spitze 29 und Schneidkante 31 mit Vorspannung an der ersten (unteren) Branche 19 gleiten.

Das insoweit beschriebene Instrument 10 arbeitet wie folgt:
Das Instrument 10 wird in der in Figur 1 und 2 veranschaulichten Position so an ein Gefäß herangeführt, dass dieses zwischen den beiden Branchen 19, 20 liegt. Der Anwender zieht dann das Griffteil 15 an den Handgriff 13 heran, womit zunächst bei noch in Rückzugsposition befindlichem Messer 21 die Branchen 19, 20 geschlossen werden. Das Gefäß wird somit zwischen der Elektrodenfläche 52 der ersten Branche 19 und der Elektrodenfläche 53 der zweiten Branche 20 gefasst und so weit abgequetscht, dass sich die einander gegenüber liegenden Gefäßwände berühren. Über den Betätigungsknopf 16 kann der Bediener nun die Aktivierung der Elektrodenflächen 52, 53 veranlassen, wobei ein zur Koagulation des Gefäßes dienender Strom zu den Branchen 19, 20 geleitet wird. Es kann sich dabei zum Beispiel um eine hochfrequente Wechselspannung handeln. Eine an dem distalen Ende einer der Branchen, zum Beispiel der Branche 20, vorgesehene Nase oder ein sonstiger Vorsprung 54 stellt dabei sicher, dass zwischen den Branchen 19, 20 ein definierter Spalt 55 eingehalten wird. Der Stromfluss zwischen den Elektrodenflächen 52, 53 läuft vollständig über das dazwischen gefasste biologische Material und koaguliert dieses. Das Messer 21 befindet sich dabei, wie Figur 5 zeigt, in Rückzugsposition.

Will der Anwender nun das koagulierte Gefäß durchtrennen, bewegt er das Griffteil 15 gegebenenfalls unter Überwindung eines erhöhten Widerstands weiter gegen den Handgriff 13, woraufhin das Messer 21, wie aus Figur 6 ersichtlich, in den Schlitz 24 der Branche 20 eingeschoben wird. Der Vertikalstellmechanismus 50 hält die Spitze 29 und die Kante 31 dabei noch schwebend über der Elektrodenfläche 52 der Branche 19. Im weiteren Verlauf der Vorwärtsbewegung senken sich die Schneidkante 31 und die Spitze 29 jedoch auf die Elektrodenfläche 52 ab, wie es die Figuren 6 und 7 veranschaulichen. Das Messer 21 wird nun von dem Antriebselement 22 bis zu dem Vorsprung 54 hin vorgeschoben und durchtrennt das koagulierte Gefäß. Dabei unterbricht der Isolator 40 jeden Strompfad zwischen dem Messer 21 und der zweiten Elektrodenfläche 52 sowie der Branche 20 und stellt somit sicher, dass das Messer 21 auch dann, wenn es etwas vorzeitig in den Schlitz 24 gelangt, keinen nennenswerten Teil des zur Koagulation dienenden Stroms übernimmt. Dies eröffnet letztendlich sogar die Möglichkeit der Gefäßdurchtrennung, während der Endphase der Koagulation der zwischen den Branchen 19, 29 festgeklemmten Gefäßenden. Damit kann eine Verkürzung der Operationszeit erreicht werden. Wesentlich aber ist, dass eine Erhöhung der Koagulationssicherheit zu verzeichnen ist. Auch wenn das Messer 21 vorzeitig betätigt wird, beeinflusst dies die Qualität des Koagulationsergebnisses nicht.

Es sei darauf hingewiesen, dass der Isolator 40 auch das gesamte Antriebselement 22 einschließen kann. In diesem Fall können das Messer 21 und das Antriebselement 22 einstückig ausgebildet oder anderweitig miteinander verbunden sein.

Außerdem wird darauf hingewiesen, dass das Antriebselement auch ganz oder teilweise aus einem elektrisch nichtleitenden Material ausgebildet sein kann. In diesem Fall kann der Isolator 40 ein Bestandteil des Antriebselements 22 sein. Z.B. kann das distale Ende des Antriebselements 22 den Isolator 40 bilden.

Bei einem Instrument 10 zur Koagulation und Fusion sowie zur Durchtrennung von Gefäßen sind zwei Branchen 19, 20 vorgesehen, zwischen denen ein Gefäß zu fassen und zu fusionieren ist. Ein Messer 21 zur Durchtrennung des koagulierten und fusionierten Gefäßes ist mit einem Isolator 40 versehen, der das Messer 21 gegen mindestens eine der Elektroden bzw. Branchen 19, 20 isoliert. Andererseits ist das Messer 21 vorzugsweise mit mindestens einer der Elektroden bzw. Branchen 19, 20 in mechanischem Kontakt, so dass eine sichere Trennung des fusionierten biologischen Gewebes bzw. Gefäßes erreicht wird. Dieses Konzept kann zur Erhöhung der Operationssicherheit und zur Miniaturisierung des Werkzeugs 18 hin zu kleineren Abmessungen genutzt werden.

### Bezugszeichenliste:

- 10: Instrument
- 11: Schaft
- 12: Gehäuse
- 13: Handgriff
- 14: Betätigungselemente
- 15: Griffteil
- 16: Betätigungsknopf- oder -hebel
- 17: Kraftübertragungsmittel
- 18: Werkzeug
- 19: erste Branche
- 20: zweite (obere) Branche
- 21: Messer
- 22: Antriebselement für 21
- 23: Raum zwischen den Branchen 19 und 20
- 24: Schlitz in 20
- 25: Schneidnase von 21
- 26: Schaft
- 27, 28: Flachseiten von 26
- α, β: Winkel
- 29: Spitze von 25
- 30: Frontschneidkante von 25
- 31: Schneidkante an Unterseite von 25
- 32: rückseitige Kante von 26
- 33: Oberseite von 26
- 34: stirnseitige Kante von 22
- 35: Parallelspalt
- 36: Oberseite von 22
- 37: untere Kante von 26
- 38: Stufe der unteren Kante von 26
- 39: Öffnung in 26
- 40: Isolator
- 41, 42: Abschnitte
- 44: Isolatorsporn
- 45: distales Ende des Messers 21
- 46: Freiraum
- 47: Öffnung in 22
- 48: Stufe an 40
- 49: Stufe an 19
- 50: Vertikalstellmechanismus
- 51: Positionierungs-Ausnehmungen in 40
- 52: Elektrodenfläche von 19
- 53: Elektrodenfläche von 20
- 54: Vorsprung
- 55: Spalt zwischen den Branchen 19 und 20
- L: Längsrichtung

## Patentansprüche

1. Instrument (10) zur Gefäßfusion und Trennung,
mit einem länglichen Schaft (11), der sich von einem Gehäuse (12) weg erstreckt und an seinem distalen Ende ein Werkzeug (18) trägt,
mit einer ersten Branche (19), die zu dem Werkzeug gehört und eine erste Elektrodenfläche (52) aufweist,
mit einer zweiten Brache (20), die zu dem Werkzeug gehört und auf die erste Elektrodenfläche (52) zu und von dieser weg beweglich gelagert ist, wobei die zweite Branche (20) eine Elektrodenfläche (53) und einen Längsschlitz (24) aufweist,
mit einem Messer (21), das zu dem Werkzeug (18) gehört und aus einem elektrisch leitfähigen Material besteht sowie relativ zu dem Schaft (11) beweglich angeordnet und in den Längsschlitz (24) hineinragend oder in diesen einfahrbar angeordnet ist, mit einem Antriebselement (22), das sich von dem Gehäuse (12) durch den Schaft (11) zu dem Messer (21) erstreckt
**dadurch gekennzeichnet, dass** das Messer (21) und das Antriebselement (22) zwischeneinander einen Spalt (35) festlegen und dass das Instrument einen Isolator (40) umfasst, der das Messer (21) und das Antriebselement (22) miteinander verbindend angeordnet ist.

2. Instrument nach Anspruch 1, **dadurch gekennzeichnet, dass** die erste Elektrodenfläche (52) eine Messergleitfläche ist.

3. Instrument nach einem der vorstehenden Ansprüche, **dadurch gekennzeichnet, dass** dem Messer (21) ein Vertikalstellmechanismus (50) zugeordnet ist, um das Messer (21) in Abhängigkeit von seiner Längsbewegung quer zu der Längsbewegung zu verstellen, wobei der Vertikalstellmechanismus (50) dazu eingerichtet ist, das Messer (21) bei einer entlang der ersten Branche (19) gerichteten Vorschubbewegung senkrecht zu der Vorschubbewegung auf die erste Elektrodenfläche (52) hin und bei einer entlang der ersten Branche (19) gerichteten Rückzugsbewegung senkrecht zu der Rückzugsbewegung von der ersten Elektrodenfläche (52) weg zu bewegen.

4. Instrument nach einem der vorstehenden Ansprüche, **dadurch gekennzeichnet, dass** die zweite Branche (20) durch einen massiven Metallkörper gebildet ist und an ihrer der Elektrodenfläche (52) der ersten Branche (19) zugewandten Seite eine der Form der Elektrodenfläche (52) folgende Form aufweist.

5. Instrument nach einem der vorstehenden Ansprüche, **dadurch gekennzeichnet, dass** mindestens eine der Branchen (19, 20) einen Vorsprung (54) aufweist, um bei geschlossenen Branchen (19, 20) des Instruments (10) zwischen der Elektrodenfläche (52) und der Elektrodenfläche (53) der zweiten Branche (20) einen Spalt (55) festzulegen.

6. Instrument nach einem der vorstehenden Ansprüche, **dadurch gekennzeichnet, dass** das Antriebselement (22) elektrisch leitend ausgebildet ist.

7. Instrument nach einem der vorstehenden Ansprüche, **dadurch gekennzeichnet, dass** das Messer (21) als Lamelle mit zwei flachen seitlichen Flanken (27, 28) ausgebildet ist.

8. Instrument nach einem der vorstehenden Ansprüche, **dadurch gekennzeichnet, dass** das Messer (21) eine Schneidnase (25) und einen Schaft (26) aufweist.

9. Instrument nach Anspruch 8, **dadurch gekennzeichnet, dass** die Schneidnase (25) eine der ersten Elektrodenfläche (52) zugewandte und parallel zu dieser angeordnete geschärfte Schneidkante (31) sowie eine stirnseitige geschärfte Schneidkante (30) aufweist, die in einem Winkel zu der Bewegungsrichtung des Messers (21) orientiert ist.

10. Instrument nach einem Anspruch 7, **dadurch gekennzeichnet, dass** sich der Schaft (26) des Messers (21) in den Isolator (40) erstreckt.

11. Instrument nach einem der vorstehenden Ansprüche, **dadurch gekennzeichnet, dass** der Isolator (40) einen Isolatorsporn (44) aufweist, der oberhalb einer Schneidnase (25) des Messers (21) angeordnet ist.

12. Instrument nach Anspruch 10, **dadurch gekennzeichnet, dass** der Schaft (26) wenigstens eine Öffnung (39) aufweist, die von dem Isolator (40) durchsetzt ist, und dass das Antriebselement (22) mindestens eine Öffnung (47) aufweist, die von dem Isolator (40) durchsetzt ist.

13. Instrument nach einem der vorstehenden Ansprüche, **dadurch gekennzeichnet, dass** sich das Antriebselement (22) in den Isolator (40) erstreckt.

14. Instrument nach einem der vorstehenden Ansprüche, **dadurch gekennzeichnet, dass** der Spalt (35) zu der Bewegungsrichtung des Messers (21) schräg orientiert ist.

## Claims

1. Instrument (18) for vessel fusion and separation,
with an elongate shank (11) extending away from the housing (12) and carrying a tool (18) at its distal end,
with a first branch (19) which belongs to the tool and comprises a first electrode face (52),
with a second branch (20) which belongs to the tool and is mounted so as to be movable towards the first electrode face (52) and away from same, wherein the second branch (20) has an electrode face (53) and a longitudinal slot (24),
with a cutter (21) which belongs to the tool (18) and consists of an electrically conductive material, and is arranged movably relative to the shank (11) and protrudes into the longitudinal slot (24) or can be inserted in same,
with a drive element (22) extending from the housing (12) through the shank (11) to the cutter (21),
**characterised in that** the cutter (21) and the drive element (22) establish a gap (35) between them, and that the instrument comprises an isolator (40) which is arranged so as to connect the cutter (21) and the drive element (22) together.

2. Instrument according to claim 1, **characterised in that** the first electrode face (52) is a cutter slide face.

3. Instrument according to any of the preceding claims, **characterised in that** a vertical adjustment mechanism (50) is assigned to the cutter (21) in order to adjust the cutter (21) transversely to its longitudinal movement, depending on the longitudinal movement, wherein the vertical adjustment mechanism (50) is configured to move the cutter (21), on an advance movement oriented along the first branch (19), perpendicularly to the advance movement towards the first electrode face (52), and on a return movement oriented along the first branch (19), perpendicularly to the return movement away from the first electrode face (52).

4. Instrument according to any of the preceding claims, **characterised in that** the second branch (20) is formed by a solid metal body and, on its side facing the electrode face (52) of the first branch (19), has a form following the form of the electrode face (52).

5. Instrument according to any of the preceding claims, **characterised in that** at least one of the branches (19, 20) has a protrusion (54) in order to establish a gap (55) between the electrode face (52) and the electrode face (53) of the second branch (20) when the branches (19, 20) of the instrument (10) are closed.

6. Instrument according to any of the preceding claims, **characterised in that** the drive element (22) is formed so as to be electrically conductive.

7. Instrument according to any of the preceding claims, **characterised in that** the cutter (21) is formed as a blade with two flat lateral flanks (27, 28).

8. Instrument according to any of the preceding claims, **characterised in that** the cutter (21) has a cutting lug (25) and a shaft (26).

9. Instrument according to claim 8, **characterised in that** the cutting lug (25) has a sharpened cutting edge (31) facing the first electrode face (52) and parallel thereto, and a sharpened cutting edge (30) on the end face which is oriented at an angle to the movement direction of the cutter (21).

10. Instrument according to claim 7, **characterised in that** the shaft (26) of the cutter (21) extends into the isolator (40).

11. Instrument according to any of the preceding claims, **characterised in that** the isolator (40) has an isolator nose (44) which is arranged above a cutting lug (25) of the cutter (21).

12. Instrument according to claim 10, **characterised in that** the shaft (26) has at least one opening (39) through which the isolator (40) passes, and that the drive element (22) has at least one opening (47) through which the isolator (40) passes.

13. Instrument according to any of the preceding claims, **characterised in that** the drive element (22) extends into the isolator (40).

14. Instrument according to any of the preceding claims, **characterised in that** the gap (35) is oriented obliquely to the movement direction of the cutter (21).

## Revendications

1. Instrument (10) de fusion de vaisseaux et de séparation, comprenant une tige (11) allongée qui s'étend à partir d'un boîtier (12) et porte un outil (18) à son extrémité distale,
comprenant un premier mors (19) qui fait partie de l'outil et présente une première surface d'électrode (52),
comprenant un deuxième mors (20) qui fait partie de l'outil et est monté de façon à pouvoir être déplacé en direction de la première surface d'électrode (52) et en être éloigné, le deuxième mors (20) présentant une surface d'électrode (53) et une fente longitudinale (24),
comprenant une lame (21) qui fait partie de l'outil (18) et est constituée d'un matériau conducteur électrique et est disposée de manière à pouvoir être déplacée par rapport à la tige (11) et de manière à pénétrer dans la fente longitudinale (24) ou pouvoir être engagée dans celle-ci,
comprenant un élément d'entraînement (22) qui s'étend du boîtier (12) jusqu'à la lame (21), en passant dans la tige (11),
**caractérisé en ce que** la lame (21) et l'élément d'entraînement (22) définissent entre eux un interstice (35), et **en ce que** l'instrument comprend un isolateur (40).

2. Instrument selon la revendication 1, **caractérisé en ce que** la première surface d'électrode (52) est une surface de glissement de lame.

3. Instrument selon l'une des revendications précédentes, **caractérisé en ce qu'**un mécanisme de réglage vertical (50) est associé à la lame (21) aux fins de déplacer la lame (21) transversalement au mouvement longitudinal, en fonction de son mouvement longitudinal, le mécanisme de réglage vertical (50) étant conçu pour déplacer la lame (21) en direction de la première surface d'électrode (52), perpendiculairement au mouvement d'avance, lors d'un mouvement d'avance orienté le long du premier mors (19), et de l'éloigner de la première surface d'électrode (52), perpendiculairement au mouvement de retrait, lors d'un mouvement de retrait orienté le long du premier mors (19).

4. Instrument selon l'une des revendications précédentes, **caractérisé en ce que** le deuxième mors (20) est constitué d'un corps métallique massif et présente, sur son côté tourné vers la surface d'électrode (52) du premier mors (19), une forme adaptée à la forme de la surface d'électrode (52).

5. Instrument selon l'une des revendications précédentes, **caractérisé en ce qu'**au moins un des mors (19, 20) présente une saillie (54) qui est destinée à définir un espace (55) entre la surface d'électrode (52) et la surface d'électrode (53) du deuxième mors (20), lorsque les mors (19, 20) de l'instrument (10) sont fermés.

6. Instrument selon l'une des revendications précédentes, **caractérisé en ce que** l'élément d'entraînement (22) est réalisé de manière à être conducteur électrique.

7. Instrument selon l'une des revendications précédentes, **caractérisé en ce que** la lame (21) est réalisée sous forme de lamelle dotée de deux flancs (27, 28) latéraux plats.

8. Instrument selon l'une des revendications précédentes, **caractérisé en ce que** la lame (21) présente une dent de coupe (25) et un corps (26).

9. Instrument selon la revendication 8, **caractérisé en ce que** la dent de coupe (25) présente une arête de coupe (31) affûtée qui est tournée vers la première surface d'électrode (52) et est disposée parallèlement à celle-ci, ainsi qu'une arête de coupe (30) affûtée qui est située côté frontal et est orientée pour former un angle par rapport à la direction de déplacement de la lame (21).

10. Instrument selon une revendication 7, **caractérisé en ce que** le corps (26) de la lame (22) s'étend dans l'isolateur (40).

11. Instrument selon l'une des revendications précédentes, **caractérisé en ce que** l'isolateur (40) présente un talon d'isolateur (44) qui est disposé au-dessus d'une dent de coupe (25) de la lame (21).

12. Instrument selon la revendication 10, **caractérisé en ce que** le corps (26) présente au moins une ouverture (39) qui est traversée par l'isolateur (40), et **en ce que** l'élément d'entraînement (22) présente au moins une ouverture (47) qui est traversée par l'isolateur (40).

13. Instrument selon l'une des revendications précédentes, **caractérisé en ce que** l'élément d'entraînement (22) s'étend dans l'isolateur (40).

14. Instrument selon l'une des revendications précédentes, **caractérisé en ce que** l'interstice (35) est orienté en biais par rapport à la direction de mouvement de la lame (21).
